Europäisches Patentamt

European Patent Office

Office européen des brevets

(19)

(11) Veröffentlichungsnummer: **0 236 827**
**B1**

(12) **EUROPÄISCHE PATENTSCHRIFT**

(45) Veröffentlichungstag der Patentschrift: 23.05.90

(51) Int. Cl.⁵: **A 61 K 7/16**

(21) Anmeldenummer: **87102496.4**

(22) Anmeldetag: **21.02.87**

(54) Zahnpasta.

(30) Priorität: **07.03.86 DE 3607480**

(43) Veröffentlichungstag der Anmeldung:
**16.09.87 Patentblatt 87/38**

(45) Bekanntmachung des Hinweises auf die
Patenterteilung:
**23.05.90 Patentblatt 90/21**

(84) Benannte Vertragsstaaten:
**AT BE CH DE ES FR GB GR IT LI LU NL SE**

(56) Entgegenhaltungen:
**EP-A-0 059 012**
**EP-A-0 097 476**
**DE-A-2 255 177**
**US-A-3 488 419**

(73) Patentinhaber: **Blendax GmbH**
**Rheinallee 88**
**D-6500 Mainz 1 (DE)**

(72) Erfinder: **Harth, Helmut, Dr.**
**Lilienweg 24**
**D-6500 Mainz 21 (DE)**

Courier Press, Leamington Spa, England.

EP 0 236 827 B1

# EP 0 236 827 B1

**Beschreibung**

Die vorliegende Erfindung betrifft eine Zahnpasta, die eine die Zahnsteinbildung reduzierende Wirkung aufweist.

Es sind bereits zahlreiche Wirkstoffe zum Einsatz in Zahn- und Mundpflegemitteln vorgeschlagen worden, die eine die Bildung von Zahnstein verringernde Wirksamkeit aufweisen sollen.

Eine in diesem Zusammenhang bereits umfangreich untersuchte Substanz ist die Ethan-1-hydroxy-1,1-diphosphonsäure (EHDP), deren Verwendung als zahnsteinhemmendes Mittel erstmals in der FR—A 1 514 194 vorgeschlagen wurde.

Von diesem Vorschlag wird zwischenzeitlich auch kommerziell Gebrauch gemacht, da eine diesen Wirkstoff enthaltende Zahnpastaseit längerem auf dem Markt angeboten wird.

Die mit einer solchen Zahnpasta erzielbare Zahnsteinreduktion liegt jedoch unter 30%.

Unter den zahlreichen weiteren für diesen Zweck vorgeschlagenen Komplexbildern ist noch auf die Alkylendiaminotetra(methylenphosphonsäuren) und deren Salze hinzuweisen, deren Verwendung als zahnsteinhemmende Mittel Gegenstand der DE—A 2 255 177 ist.

Es ist bekannt, daß solche die Zahnsteinbildung verringernde Wirkstoffe in Zahnpasten nur in Kombination mit keine Calciumionen abgebenden Poliermitteln zum Einsatz gelangen sollen, wie bereits aus der obengenannten FR—A 1 514 194 hervorgeht.

Bevorzugte Putzkörper in solchen Komplexbildner enthaltenden Zusammensetzungen sind die verschiedenen Siliciumdioxide bzw. Silikagele, wir es beispielsweise auch in der DE—A 2 310 771 vorgeschlagen wird.

Eine weitere Gruppe von Substanzen, die sowohl in vitro als auch in vivo eine die Zahnsteinbildung vermindernde Wirkung aufweisen, sind Tetraalkalipyrophosphate, insbesondere Tetranatrium- und Tetrakaliumpyrophosphat, vgl. W. W. Briner, M. D. Francis, Calc. Tiss. Res. 11 (1973), 10 bis 22.

Von der Erkenntnis dieser wissenschaftlichen Veröffentlichung macht auch die EP—A 97 476 Gebrauch, die Zahn- und Mundpflegemittel zum Gegenstand hat, die, in an sich üblicher Grundlage (selbstverständlich in Abwesenheit von Calciumionen abspaltenden Verbindungen), Dialkali- bzw. Tetraalkalipyrophosphate enthalten.

Derartige Zahnpasten haben in klinischen Untersuchungen eine durchschnittliche Reduktion der Zahnsteinbildung zwischen etwa 26 und etwa 35% erzielt (vgl. J. Amer. Dental Assoc. 110 (1985), 737 bis 738 und J. Dental Res. *64* (1985), 1159 bis 1162).

Die durch die bekannten, oben beschriebenen Zahnpasten erzielbare Reduktion der Zahnsteinbildung erscheint somit nicht ausreichend.

Es wurde nun gefunden, daß sich eine wesentlich höhere Reduktion der Zahnsteinbildung erzielen läßt, wenn man als zahnsteinverhinderndes Mittel in einer Zahnpasta ein Gemisch aus mindestens einem Dialkali- oder Tetraalkalipyrophosphat und mindestens einer Substanz der Gruppe Ethan-1-hydroxy-1,1-diphosphonsäure bzw. deren Salzen und/oder Alkylendiaminotetra(methylenphosphonsäuren) bzw. deren Salzen einsetzt.

Es war überrachend und nicht vorhersehbar, daß sich durch die Kombination dieser Wirkstoffe eine Wirkungssteigerung erzielen läßt, da einerseits bekannt ist, daß eine Konzentrationserhöhung der einzelnen Wirkstoffe zu keiner proportionalen Aktivitätserhöhung führt und darüber hinaus auch für den Zahnschmelz schädlich sein kann, da sie möglicherweise zu einer Behinderung der Remineralisation führen kann, andererseits jedoch bisher auch durch die Verwendung anderer Kombinationen aus bekannten komplexbildenden Wirkstoffen eine erwünschte Aktivitätserhöhung nicht beobachtet werden konnte.

Es bedurfte daher einer echten erfinderischen Auswahl, unter den zahlreichen zur Verfügung stehenden Möglichkeiten gerade die eng begrenzten Substanzgruppen auszuwählen, durch deren gemeinsame Anwendung eine überraschende synergistische Wirkungssteigerung auftritt.

Diese Wirkung wurde im Rattenexperiment überzeugend bestätigt. Der Anteil an Ethan-1-hydroxy-1,1-diphosphonsäure in der erfindungsgemäßen Zahnpasta liegt dabei vorzugsweise bei etwa 0,2 bis 2,0 Gew.-% Gesamtzusammensetzung, insbesondere zwischen etwa 0,5 und 1,0 Gew.-%.

Falls die Salze, beispielsweise das Di- und Trinatriumsalz von EHDP eingesetzt werden, bezieht sich die Berechnung auf den Anteil an freier Säure.

Falls Alkylendiaminotera(methylenphosphonsäuren) oder deren Salze, vorzugsweise $C_2$—$C_8$-Alkylendiaminotetra(methylenphosphonsäuren), zum Einsatz gelangen, liegt deren Anteil zwischen etwa 0,5 und 5,0, vorzugsweise zwischen 1 und 3, insbesondere bei etwa 1,5 Gew.%, jeweils berechnet auf die Säure.

Bevorzugte Salze sind auch hier die Tetranatriumsalze.

Besonders geeignet sind die Ethylen- und Hexamethylenalkylendiaminotetra(methylenphosphonsäuren) und deren Salze.

Geeignete Alkalipyrophosphate sind insbesondere Dinatrium- und Dikalium- sowie Tetranatrium- und Tetrakaliumpyrophosphat. Der Anteil dieser Verbindungen in dem erfindungsgemäßen Gemisch liegt zwischen etwa 1 und etwa 5 Gew.-%, vorzugsweise zwischen 2 und 4, insbesondere bei etwa 2,5 bis 3,5 Gew.-%, bezogen auf die Gesamtzusammensetzung der Zahnpasta, jeweils berechnet auf das Pyrophosphation.

2

# EP 0 236 827 B1

Wie bereits ausgeführt, sollen die erfindungsgemäßen Zahnpasten keine Poliermittel enthalten, die in der Lage sind, Calciumionen in nennenswertem Umfang abzugeben.

Geeignete Poliermittel sind deshalb insbesondere verschiedene Siliciumdioxid-Modifikationen wie gefällte Silikagele, Siliciumdioxid-Xerogele und -Hydrogele, Alkalialuminiumsilikate, beispielsweise solche vom Zeolith Typ wie synthetisches Natriumaluminiumsilikat der empirischen Formel $NA_{12}(AlO_2)_{12}(SiO_2)_{12}.27H_2O$, Aluminiumoxid und Aluminiumoxidtrihydrat, unlösliches Metaphosphat, pulverförrmige Kunststoffe sowie auch hitzebehandeltes Calciumpyrophosphat.

Es können selbstverständlich auch Poliermittelgemische aus den genannten Substanzen eingesetzt werden, beispielsweise ein Gemisch aus a-Aluminiumoxidtrihydrat und/oder unlöslichem Alkalimetaphosphat und synthetischem Zeolith A im Verhältnis von etwa 1:1.

Der Poliermittelanteil in den erfindungsgemäßen Zahnpasten liegt vorzugsweise zwischen etwa 20 und 60 Gew.-% der Gesamtzusammensetzung.

Es ist selbstverständlich möglich, die üblichen in Zahnpasten eingesetzten oberflächenaktiven Verbindungen in Mengen bis zu etwa 2,5 Gew.-% der Gesamtzusammensetzung zu verwenden.

Solche synthetischen oberflächenaktiven Stoffe sind beispielsweise Alkylsulfate, Alkylethersulfate, Olefinsulfonate, Natriumlauroylsarcosinat oder ampholytische, nichtionische oder kationaktive Verbindungen oder auch Seifen wie beispielsweise solche von Laurinsäure, Myristinsäure, Palmitinsäure, Stearinsäure oder Gemischen derselben, beispielsweise Cocosölfettsäuren oder Talgfettsäuren.

Gleiches gilt hinsichtlich der in Zahnpasten üblicherweise in Mengen zwischen etwa 10 und etwa 35 Gew.-% zum Einsatz gelangenden Feuchthaltemittel, wie Glycerin, Diolen wie 1,4-Butandiol oder 1,2-Propandiol oder Zuckeralkoholen wie Sorbit, Mannit oder Xylit und Polyglykolen mit niederen Molekulargewichten, ebenso für Verdickungsmittel, deren Mengenanteil in Zahnpasten zwischen etwa 0,25 und etwa 2,5 Gew.-% Gesamtzusammensetzung liegt.

Bevorzugte Verdickungsmittel sind Carboxymethylcellulose und deren Alkalisalze, insbesondere Natriumcarboxymethylcellulose, Hydroxyalkylcellulosen wie Hydroxymethylcellulose und Hydroxyethylcellulose, Methylcellulose, Pflanzengummen wie Tragant, Gummi arabicum, Carayagummi, Guargummi, Xanthangummi und Irish Moos, synthetische Polyelektrolyte wie die Amin- und Alkalisalze der Polyacrylsäure sowie anorganische Verdickungsmittel, beispielsweise kolloidales Magnesiumaluminiumsilikat oder disperses Siliciumoxid.

In den erfindungsgemäßen Zahnpasten können selbstverständlich auch weitere Wirkstoffe Verwendung finden. Solche sind insbesondere die bekannten kariesprophylaktischen Fluoride, vorzugsweise in einer solchen Menge, daß die Konzentration an reinem Fluor im Mittel etwa 0,05, bis etwa 1 Gew.-%, vorzugsweise 0,1 bis 0,5 Gew.-% des Mittels beträgt.

Geeignete Fluorverbindungen sind insbesondere die verschiederen Salze der Monofluorphosphorsäure wie Natrium-, Kalium-, Lithium-, Calcium- und Aluminiummono- und difluorphosphat sowie verschiedene, Fluor in ionisch gebundener Form enthaltene Fluoride, insbesondere Alkalifluoride wie Natrium-, Lithium-, Kalium- und Ammoniumfluorid, Zinnfluorid, Manganfluorid, Kupferfluorid, Zirkoniumfloride und Aluminiumflorid sowie Gemische oder Anlagerungsprodukte dieser Fluoride untereinander und mit anderen Fluorverbindungen, beispielsweise Alkalimanganfluoride.

Weitere, in den erfindungsgemäßen Zahnpasten einsetzbare Stoffe sind Mittel zur Verhinderung von Zahnbelagsbildung wie Chlorhexidinsalze, Zink- und Kupferverbindungen, Harnstoff, Hexetidin, Hesperidin, Allentoin, Azulen, etc.

Der pH-Wert der erfindungsgemäßen Zahnpasta liegt zwischen etwa 4 und etwa 10, vorzugsweise 5,5 und 9,5.

Eine Übersicht über in Zahnpasten einsetzbare Zusammensetzungen findet sich, wie überhaupt über sonstige zur Herstellung von Zahnpflegemitteln üblicherweise zum Einsatz gelangende Stoffe und die dabei angewandten Herstellungsverfahren, in dem Handbuch von M. S. Balsam und E. Sagarin, "Cosmetics — Science and Technology", 2nd Ed., Vol. 1, S. 423 bis 533 (1972), auf das hier ausdrücklich Bezug genommen wird.

Im folgenden werden einige Beispiele gegeben, die das Wesen der vorliegenden Erfindung charakterisieren:

# EP 0 236 827 B1

## Beispiel 1

| | |
|---|---|
| $\alpha$-Aluminiumoxidtrihydrat | 38,00 (Gew.-%) |
| Sorbit | 10,00 |
| Glycerin | 5,00 |
| Methylcellulose | 0,80 |
| Hydroxyethylcellulose | 0,40 |
| Natriummonofluorphosphat | 0,76 |
| Natriumfluorid | 0,11 |
| Natriumlaurylsulfat | 1,20 |
| Aromamischung | 1,00 |
| Saccharin-Natrium | 0,08 |
| Kolloidales Siliciumdioxid | 3,50 |
| Methyl-p-hydroxybenzoat, Natriumsalz | 0,25 |
| EHDP, Trinatriumsalz | 0,85 |
| Tetranatriumpyrophosphat | 3,75 |
| Allantoin | 0,25 |
| Wasser | ad 100,00 |

## Beispiel 2

| | |
|---|---|
| Gefälltes Siliciumdioxid-Gel | 23,50 (Gew.-%) |
| Siliciumdioxid-Aerogel | 2,50 |
| Glycerin | 9,00 |
| Sorbit | 17,50 |
| Natriumlaurylsulfat | 1,50 |
| Carboxymethylcellulose | 1,25 |
| Natriumfluorid | 0,25 |
| Aromamischung | 1,10 |
| Saccharin-Natrium | 0,05 |
| Kupfersulfat . $5H_2O$ | 0,25 |
| Hexetidin | 0,05 |
| Methyl-p-hydroxybenzoat, Natriumsalz | 0,20 |
| n-Propyl-p-hydroxybenzoat, Natriumsalz | 0,10 |
| Hexamethylendiaminotetra(methylenphosphon-säure), Tetranatriumsalz | 1,30 |
| Tetrakaliumpyrophosphat | 3,10 |
| Wasser | ad 100,00 |

4

EP 0 236 827 B1

<u>Beispiel 3</u>

| | | |
|---|---|---|
| Unlösliches Natriummetaphosphat | 38,00 | (Gew.-%) |
| Sorbit | 13,00 | |
| Kolloidales Siliciumdioxid | 2,80 | |
| Hydroxyethylcellulose | 1,80 | |
| Methyl-p-hydroxybenzoat | 0,10 | |
| Benzoesäure | 0,10 | |
| Saccharin-Natrium | 0,20 | |
| Aromamischung | 1,10 | |
| Fettalkoholpolyglycolether | 0,60 | |
| 1,2-Propandiol | 4,00 | |
| Titandioxid | 0,80 | |
| N,N',N'-Tri(2-hydroxyethyl)-N-octadecyl-1,3-diaminopropandihydrofluorid | 2,00 | |
| Dinatriumpyrophosphat | 2,80 | |
| Tetrakaliumpyrophosphat | 1,20 | |
| EHDP, Dinatriumsalz | 0,60 | |
| Wasser | ad 100,00 | |

<u>Beispiel 4</u>

| | | |
|---|---|---|
| Polymethylmethacrylat-Pulver (mittl. Teilchengröße 1-5 µm) | 15,00 | (Gew.-%) |
| Hitzebehandeltes ß-Calciumpyrophosphat | 15,00 | |
| Natriumcarboxymethylcellulose | 1,20 | |
| Natriumlauroylsarcosinat | 0,80 | |
| Natriumlaurylsulfat | 0,80 | |
| Natriummonofluorphosphat | 1,20 | |
| Saccharin-Natrium | 0,10 | |
| Aromamischung | 1,10 | |
| Ethylendiaminotetra(methylenphosphonsäure), Tetranatriumsalz | 1,80 | |
| Tetranatriumpyrophosphat | 3,20 | |
| EHDP, Dinatriumsalz | 0,30 | |
| Glycerin | 10,00 | |
| Sorbit | 8,00 | |
| Wasser | ad 100,00 | |

5

### Beispiel 5

| | |
|---|---|
| Silica-Xerogel | 25,00 (Gew.-%) |
| Silica-Aerogel | 2,80 |
| Sorbit | 35,00 |
| Xanthum-Gum | 1,10 |
| Natriumfluorid | 0,30 |
| EHDP, Dinatriumsalz | 0,80 |
| Dinatriumpyrophosphat | 1,00 |
| Tetranatriumpyrophosphat | 1,80 |
| Tetrakaliumpyrophosphat | 1,00 |
| Natriumlaurylsulfat | 1,30 |
| Natriumlauroylsarcosinat | 0,80 |
| Aromamischung | 1,00 |
| Saccharin-Natrium | 0,10 |
| Methyl-p-hydroxybenzoat, Natriumsalz | 0,30 |
| Farbstoff | q.s. |
| Wasser | ad 100,00 |

### Beispiel 6

| | |
|---|---|
| Calciniertes ß-Calciumpyrophosphat | 45,00 (Gew.-%) |
| Unlösliches Natriummetaphosphat | 5,00 |
| Glycerin | 5,50 |
| Sorbit | 12,50 |
| Natriumcarboxymethylcellulose | 1,20 |
| Natriumsulforicinoleat | 0,80 |
| Natriumlaurylsulfat | 1,20 |
| Ethylendiaminotetra(methylenphosponsäure), Tetranatriumsalz | 2,50 |
| Tetranatriumpyrophosphat | 2,00 |
| Dinatriumpyrophosphat | 1,20 |
| Natriummonofluorphosphat | 1,14 |
| Saccharin-Natrium | 0,11 |
| Aromagemisch | 1,00 |
| Natriumbenzoat | 0,30 |
| Methyl-p-hydroxybenzoat, Natriumsalz | 0,15 |
| Wasser | ad 100,00 |

# EP 0 236 827 B1

**Patentansprüche**

1. Zahnpasta mit die Bildung von Zahnstein reduzierender Wirkung, die im wesentlichen frei von wasserlöslichen Calciumionen liefernden Verbindungen ist, dadurch gekennzeichnet, daß sie ein Gemisch aus einem Di- und/oder Tetraalkalipyrophosphat und Ethan-1-hydroxy-1,1-diphosphonsäure bzw. deren wasserlöslichen Salzen und/oder Alkylendiaminotetra(methylenphosphonsäuren) bzw. deren wasserlöslichen Salzen enthält.

2. Zahnpasta nach Anspruch 1, dadurch gekennzeichnet, daß sie Di- und/oder Tetraalkalipyrophosphat in einer Menge von 1 bis 5 Gew.-% (berechnet auf Pyrophosphat), bezogen auf die Gesamtzusammensetzung, enthält.

3. Zahnpasta nach Anspruch 1, dadurch gekennzeichnet, daß sie die Ethan-1-hydroxy-1,1-diphosphonsäure bzw. deren Salze in einer Menge von 0,2 bis 2,0 Gew.-% (berechnet auf freie Phosphonsäure), bezogen auf die Gesamtzusammensetzung, enthält.

4. Zahnpasta nach Anspruch 1, dadurch gekennzeichnet, daß sie die Alkylendiaminotetra(methylenphosphonsäuren) bzw. deren Salze in einer Menge von 0,5 bis 5,0 Gew.-% (berechnet auf freie Phosphonsäuren), bezogen auf die Gesamtzusammensetzung, enthält.

**Revendications**

1. Pâte dentifrice ayant pour effet de réduire la formation de tartre dentaire, essentiellement dépourvue de composés donnant des ions calcium hydrosolubles, caractérisée en ce qu'elle contient un mélange constitué par un pyrophosphate di- et/ou tétraalcalin et l'acide éthane-1-hydroxy-1,1-diphosphonique ou ses sels hydrosolubles et/ou des acides alkylènediamino(méthylènephosphonique) ou leurs sels hydrosolubles.

2. Pâte dentifrice selon la revendication 1, caractérisée en ce qu'elle contient le pyrophosphate di- et/ou tétraalcalin en une quantité de 1 à 5% en poids (calculée comme pyrophosphate) par rapport à la composition totale.

3. Pâte dentifrice selon la revendication 1, caractérisée en ce qu'elle contient l'acide éthane-1-hydroxy-1,1-diphosphonique ou ses sels en une quantité 0,2 à 2,0% en poids (calculée en acide phosphonique libre) par rapport à la composition totale.

4. Pâte dentifrice selon la revendication 1, caractérisée en ce qu'elle contient les acides alkylènediamino (méthylènephosphoniques) ou leurs sels en une quantité de 0,5 à 5,0% en poids (calculée comme acides phosphoniques libres) par rapport à la composition totale.

**Claims**

1. Toothpaste reducing the formation of dental calculus, being substantially free from compounds releasing calcium ions, characterized in that it contains a mixture of a di- and/or tetraalkali pyrophosphate and ethan-1-hydroxy-1,1-diphosphonic acid and/or water-soluble salts thereof and/or alkylene diaminotetra(methylene phosphonic acids) and/or water-soluble salts thereof.

2. Toothpaste according to claim 1, characterized in that it contains di- and/or tetraalkali pyrophosphate in a quantity of 1 to 5% by weight (calculated to pyrophosphate), referred to the total toothpaste composition.

3. Toothpaste according to claim 1, characterized in that it contains ethan-1-hydroxy-1,1-diphosphonic acid and/or salts thereof in a quantity of 0,2 to 2,0% by weight (calculated to free phosphonic acid), referred to the total toothpaste composition.

4. Toothpaste according to claim 1, characterized in that it contains alkylendiaminotetra(methylene phosphonic acids) and/or salts thereof in a quantity of 0,5 to 5,0% by weight (calculated to free phosphonic acid), referred to the total composition.